(19) Europäisches Patentamt
European Patent Office
Office européen des brevets

(11) Veröffentlichungsnummer : **0 110 828**
**B1**

(12) # EUROPÄISCHE PATENTSCHRIFT

(45) Veröffentlichungstag der Patentschrift : **20.01.88**

(21) Anmeldenummer : **83810536.9**

(22) Anmeldetag : **21.11.83**

(51) Int. Cl.[4] : **G 03 C 7/26**, C 07 D401/12, C 07 D491/113, C 07 D401/04 // (C07D491/113, 319:00, 221:00)

(54) **Farbphotographisches Aufzeichnungsmaterial.**

(30) Priorität : **26.11.82 CH 6894/82**

(43) Veröffentlichungstag der Anmeldung : **13.06.84 Patentblatt 84/24**

(45) Bekanntmachung des Hinweises auf die Patenterteilung : **20.01.88 Patentblatt 88/03**

(84) Benannte Vertragsstaaten : **BE DE FR GB IT**

(56) Entgegenhaltungen :
**EP-A- 0 011 051**
**FR-A- 2 334 672**
**FR-A- 2 357 560**
**GB-A- 1 059 842**

(73) Patentinhaber : **CIBA-GEIGY AG**
**Klybeckstrasse 141**
**CH-4002 Basel (CH)**

(72) Erfinder : **Leppard, David G., Dr.**
**Route de Bourguillon 6**
**CH-1723 Marly (CH)**
Erfinder : **Rody, Jean, Dr.**
**Rütiring 82**
**CH-4125 Riehen (CH)**

**EP 0 110 828 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein farbphotographisches Aufzeichnungsmaterial, das in mindestens einer lichtempfindlichen Silberhalogenidemulsionsschicht und/oder in mindestens einer der üblichen Hilfsschichten als Stabilisator eine spezifische heterocyclische Verbindung enthält. Bei diesen Verbindungen handelt es sich um Isocyanursäurederivate oder Barbitursäurederivate, die in ihrem Molekül sowohl ein sterisch gehinderte Phenolgruppe wie eine Polyalkylpiperidingruppe enthalten.

Aus der EP-A 11 051 sind bereits Molekülkombinationen von sterisch gehinderten Phenolen mit Polyalkylpiperidinen bekannt, die als Stabilisatoren für farbphotographische Materialien verwendbar sind. Es handelt sich dabei um Polyalkylpiperidinylester von Hydroxybenzylmalonsäuren. In Weiterverfolgung dieser Forschungsarbeiten wurde gefunden, dass gewisse heterocyclische Verbindungen, die sterisch gehinderte Phenol- und Piperidingruppen enthalten, ebenfalls eine ausgezeichnete Lichtschutzwirkung für Farbphotographien besitzen und darüber hinaus auch im Dunkeln eine Stabilisierung der Farbstoffe gegenüber Veränderungen beim Lagern des Aufzeichnungsmaterials bewirken.

Gegenstand der vorliegenden Erfindung ist daher ein farbphotographisches Aufzeichnungsmaterial, das in mindestens einer lichtempfindlichen Silberhalogenidemulsionsschicht, einer Zwischenschicht, einer Bildempfangsschicht und/oder einer Schutzschicht als Stabilisator mindestens eine Verbindung der Formel I enthält,

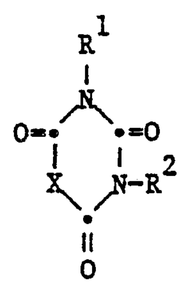

(I)

worin X eine Gruppe

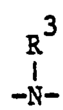

oder

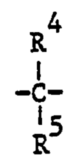

darstellt und $R^1$, $R^2$, $R^3$, $R^4$ und $R^5$ einwertige organische Reste sind, von denen mindestens einer eine sterisch gehinderte Phenolgruppe und mindestens einer eine Polyalkylpiperidingruppe enthält.

Diese Stabilisatoren sind insbesondere solche Verbindungen der Formel I, worin X eine Gruppe $—N(R^3)—$ oder $—C(R^4)(R^5)—$ darstellt, $R^1$, $R^2$ und $R^3$ unabhängig voneinander entweder

a) eine Gruppe der Formel II sind

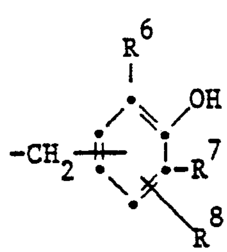

(II)

worin

$R^6$ Wasserstoff, $C_1$-$C_8$-Alkyl, $C_5$-$C_8$-Cycloalkyl, $C_7$-$C_9$-Phenylalkyl, Phenyl oder $C_7$-$C_{10}$-Alkylphenyl bedeutet,

$R^7$ $C_1$-$C_8$-Alkyl, $C_5$-$C_8$-Cycloalkyl, $C_7$-$C_9$-Phenylalkyl, Phenyl oder $C_7$-$C_{10}$-Alkylphenyl bedeutet,

$R^8$ Wasserstoff oder Methyl ist,

oder

b) eine Gruppe der Formel III oder IV sind,

$$—C_mH_{2m}—CO—Y—D \qquad (III)$$

$$—C_mH_{2m}—CO—O—C_nH_{2n}—E \qquad (IV)$$

worin m 1-4 und n 2-5 ist,

Y —O— oder $—NR^9—$ ist und $R^9$ Wasserstoff, $C_1$-$C_{12}$-Alkyl, $C_5$-$C_{12}$-Cycloalkyl, $C_3$-$C_{12}$-Alkoxyalkyl, $C_4$-$C_{12}$-Dialkylaminoalkyl oder eine Gruppe der Formel

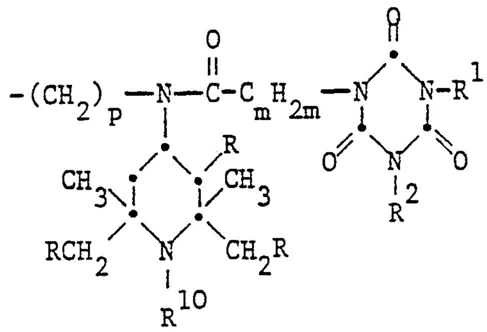

beudeutet, worin p 2-12 ist, oder $R^9$ gleich D ist,

D eine Gruppe der Formel V, VI oder VII ist,

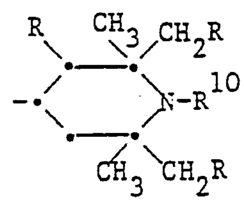

(V)

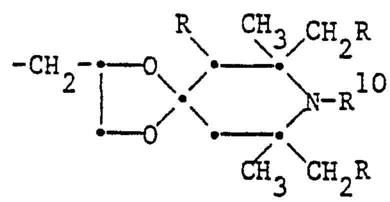

(VI)

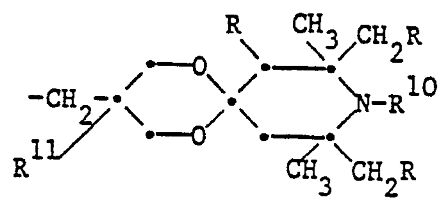

(VII)

und E eine Gruppe der Formel VIII ist,

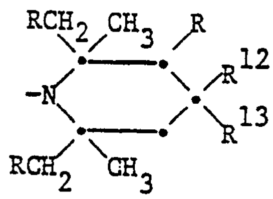

(VIII)

worin

R Wasserstoff oder Methyl ist,

$R^{10}$ Hydroxyl, $C_1$-$C_{12}$-Alkyl, $C_3$-$C_6$-Alkenyl, $C_3$-$C_4$-Alkinyl, $C_7$-$C_{12}$-Phenylalkyl, Glycidyl, durch Halogen, —CN, $—COOR^{15}$ oder $—CON(R^{16})(R^{17})$ substituiertes $C_1$-$C_4$-Alkyl, eine Gruppe $—CO—R^{18}$, $—CO—OR^{15}$, $—CO—N(R^{16})(R^{17})$, $—CH_2—CH(R^{19})—OR^{20}$, $—SO—R^{21}$, $—SO_2—R^{21}$, $—OR^{15}$ oder $—OOC—R^{18}$ bedeutet,

$R^{11}$ Methyl oder Ethyl ist,

$R^{12}$ Wasserstoff, $—OR^{22}$, $—OOC—R^{18}$, oder $—N(R^9)—CO—R^{18}$ und

$R^{13}$ Wasserstoff, —CN, —$COOR^{15}$ oder —$CONH_2$ bedeuten oder
$R^{12}$ und $R^{13}$ zusammen eine Gruppe der folgenden Formeln bilden,

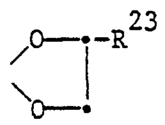

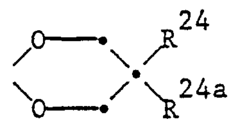

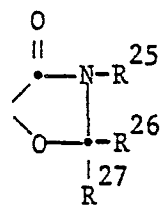

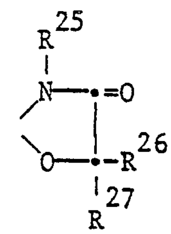

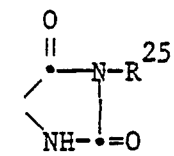

$R^{15}$ $C_1$-$C_{12}$-Alkyl, Allyl, Benzyl oder Cyclohexyl bedeutet,
$R^{16}$ $C_1$-$C_{12}$-Alkyl, Allyl, Cyclohexyl, Benzyl oder Phenyl, und
$R^{17}$ Wasserstoff oder $C_1$-$C_8$-Alkyl bedeuten oder
$R^{16}$ und $R^{17}$ zusammen mit dem N-Atom einen 5- oder 6-gliedrigen heterocyclischen Ring bilden,
$R^{18}$ Wasserstoff, $C_1$-$C_{12}$-Alkyl, $C_2$-$C_6$-Alkenyl, Chlormethyl, $C_5$-$C_{12}$-Cycloalkyl, $C_7$-$C_{12}$-Phenylalkyl, Phenyl, $C_7$-$C_{10}$-Alkylphenyl oder durch 1 oder 2 $C_1$-$C_4$-Alkylgruppe und eine Hydroxygruppe substituiertes Phenyl, Phenylmethyl oder Phenylethyl bedeutet,
$R^{19}$ Wasserstoff, $C_1$-$C_4$-Alkyl, $C_2$-$C_{13}$-Alkoxymethyl, Phenyl oder Phenoxymethyl beudeutet,
$R^{20}$ Wasserstoff, $C_1$-$C_{12}$-Alkyl, —CO—$R^{18}$ oder —CO—N($R^{16}$)($R^{17}$) bedeutet,
$R^{21}$ $C_1$-$C_{12}$-Alkyl, Phenyl oder $C_7$-$C_{18}$-Alkylaryl bedeutet,
$R^{22}$ Wasserstoff, $C_1$-$C_{12}$-Alkyl, Allyl oder Benzyl ist,
$R^{23}$ Wasserstoff, Methyl oder Ethyl ist,
$R^{24}$ und $R^{24a}$ unabhängig voneinander H oder $C_1$-$C_4$-Alkyl bedeuten,
$R^{25}$ Wasserstoff, $C_1$-$C_{12}$-Alkyl, $C_3$-$C_5$-Alkenyl oder $C_7$-$C_{12}$-Phenylalkyl bedeutet,
$R^{26}$ Wasserstoff, $C_1$-$C_{12}$-Alkyl, $C_3$-$C_8$-Cycloalkyl oder Benzyl und
$R^{27}$ $C_1$-$C_{12}$-Alkyl, $C_5$-$C_8$-Cycloalkyl oder Phenyl ist, oder
$R^{26}$ und $R^{27}$ zusammen mit dem C-Atom, an das sie gebunden sind, einen $C_5$-$C_{12}$-Cycloalkan- oder Alkylcycloalkanring bilden,
oder
c) Wasserstoff, $C_1$-$C_{18}$-Alkyl, $C_3$-$C_6$-Alkenyl, $C_7$-$C_{12}$-Phenylalkyl, $C_3$-$C_{12}$-Alkoxyalkyl oder $C_3$-$C_{14}$-Alkoxycarbonylalkyl darstellen,
$R^4$ Wasserstoff, $C_1$-$C_{18}$-Alkyl, $C_5$-$C_8$-Cycloalkyl, eine Gruppe der Formel II, eine Gruppe der Formel III oder IV oder eine Gruppe —$C_mH_{2m}$—COO($C_1$-$C_4$-Alkyl) bedeutet und
$R^5$ Wasserstoff, $C_1$-$C_{18}$-Alkyl, $C_5$-$C_8$-Cycloalkyl, Phenyl, $C_7$-$C_9$-Phenylalkyl, eine Gruppe der Formel II, eine Gruppe der Formel III oder IV oder eine Gruppe der Formel V bedeutet,
wobei von den Resten $R^1$, $R^2$, $R^3$, $R^4$, $R^5$ mindestens einer eine sterisch gehinderte Phenolgruppe enthält und mindestens einer eine Polyalkylpiperidingruppe enthält.

Darin können $R^{19}$, $R^{24}$ und $R^{24a}$ $C_1$-$C_4$-Alkyl sein wie z. B. Methyl, Ethyl, Propyl, Isopropyl, n-Butyl, sec.-Butyl oder tert.-Butyl. $R^6$, $R^7$ und $R^{17}$ als $C_1$-$C_8$-Alkyl können darüber hinaus auch z. B. Isoamyl, n-Hexyl, 2-Ethylbutyl, n-Octyl oder 1,1,3,3-Tetramethylbutyl sein. $R^9$, $R^{10}$, $R^{15}$, $R^{16}$, $R^{18}$, $R^{20}$, $R^{21}$, $R^{22}$, $R^{25}$ und $R^{26}$ als $C_1$-$C_{12}$-Alkyl können darüber hinaus auch z. B. Nonyl, Decyl oder Dodecyl sein. $R^1$, $R^2$, $R^3$, $R^4$ und $R^5$ als $C_1$-$C_{18}$-Alkyl können darüber hinaus auch z. B. Tetradecyl, Hexadecyl oder Octadecyl sein.

$R^1$, $R^2$, $R^3$ und $R^9$ als $C_3$-$C_{12}$-Alkoxyalkyl können z. B. 2-Methoxyethyl, 2-Ethoxyethyl, 2-Isopropoxyethyl, 3-Methoxypropyl, 2-Butoxyethyl, 2-Butoxypropyl oder 2-Hexyloxyethyl sein. $R^{19}$ als $C_2$-$C_{13}$-Alkoxymethyl kann z. B. Methoxy-, Ethoxy-, Butoxy-, 2-Ethylbutyloxy-, Hexyloxy- oder Dodecyloxymethyl sein.

$R^9$ als $C_4$-$C_{12}$-Dialkylaminoalkyl kann z. B. 2-Dimethylaminoethyl, 3-Diethylaminopropyl, 2-Dipropylaminoethyl oder 3-Dibutylaminopropyl sein.

$R^1$, $R^2$ und $R^3$ als $C_3$-$C_{14}$-Alkoxycarbonylalkyl können z. B. Alkoxycarbonylmethyl, 2-Alkoxycarbonylethyl oder 2-Alkoxycarbonylpropyl sein.

$R^1$, $R^2$, $R^3$, $R^{10}$, $R^{18}$ und $R^{25}$ als $C_3$-$C_6$-Alkenyl können insbesondere $C_2$-$C_5$-Alkenyl-methyl sein, wie z. B. Allyl, Methallyl, 3-Methylallyl oder 3,3-Dimethylallyl. $R^{10}$ als $C_3$-$C_4$-Alkinyl kann z. B. Propargyl oder 3-Methylpropargyl sein.

$R^4$, $R^5$, $R^6$, $R^7$, $R^{26}$ und $R^{27}$ als $C_5$-$C_8$-Cycloalkyl können z. B. Cyclopentyl, Cyclohexyl, Methylcyclohexyl, Dimethylcyclohexyl, Cycloheptyl oder Cyclooctyl sein. $R^9$ und $R^{18}$ als $C_5$-$C_{12}$-Cycloalkyl können darüber hinaus auch z. B. Cyclodecyl oder Cyclododecyl sein.

Wenn $R^{26}$ und $R^{27}$ zusammen mit dem C-Atom, an das sie gebunden sind, einen Cycloalkan- oder Alkylcycloalkanring bilden, so kann dies z. B. ein Cyclopentan-, Cyclohexan-, Methylcyclohexan-, Dimethylcyclohexan-, Cycloheptan-, Cyclooctan- oder Cyclododecanring sein.

$R^5$, $R^6$ und $R^7$ als $C_7$-$C_9$-Phenylalkyl können z. B. Benzyl, 2-Phenylethyl, 3-Phenylpropyl oder 1-Phenylisopropyl sein. $R^1$, $R^2$, $R^3$, $R^{10}$, $R^{18}$ und $R^{25}$ als $C_7$-$C_{12}$-Phenylalkyl können darüber hinaus auch z. B. 3-Phenylbutyl oder 6-Phenylhexyl sein.

$R^6$, $R^7$ und $R^{18}$ als $C_7$-$C_{10}$-Alkylphenyl können z. B. Tolyl, Xylyl, Ethylphenyl, Isopropylphenyl oder tert-Butylphenyl sein. $R^{21}$ als $C_7$-$C_{18}$-Alkylaryl kann darüber hinaus auch z. B. Methylnaphthyl, Butylnaphthyl, Nonylphenyl oder Dopecylphenyl sein.

$R^{16}$ und $R^{17}$ können jeweils zusammen mit dem N-Atom, an das sie gebunden sind, einen 5- oder 6-gliedrigen heterocyclischen Ring bilden. Dies kann z. B. ein Pyrrolidin-, Piperidin-, Morpholin- oder 4-Methylpiperazinring sein.

Im phenolischen Rest der Formel II kann die Hydroxylgruppe in meta- oder para-Stellung zur $CH_2$-Gruppe sein, bevorzugt ist sie in para-Stellung. Wenn die $CH_2$-Gruppe in p-Stellung zur Hydroxylgruppe ist, so ist $R^8$ in m-Stellung. Wenn die $CH_2$-Gruppe in m-Stellung ist, so ist $R^8$ in p-Stellung zur Hydroxylgruppe.

Bevorzugt sind Stabilisatoren der Formel I, worin X eine Gruppe $—N(R^3)_5—$ oder $—C(R^4)(R^5)—$ darstellt und von den Resten $R^1$, $R^2$, $R^3$, $R^4$ und $R^5$ mindestens einer eine sterisch gehinderte Phenolgruppe und mindestens einer eine N-substituierte 2,2,6,6-Tetramethylpiperidingruppe enthält.

Besonders bevorzugt sind Stabilisatoren der Formel I, worin X eine Gruppe $—N(R^3)—$ oder $—C(R^4)(R^5)—$ darstellt und $R^1$, $R^2$ und $R^3$ unabhängig voneinander entweder

a) eine Gruppe der Formel IIa oder IIb ist,

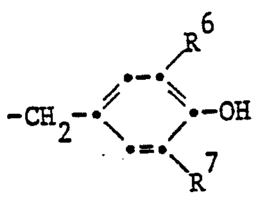
(IIa)

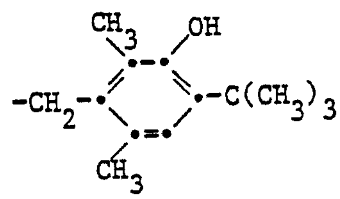
(IIb)

worin $R^6$ Wasserstoff, $C_1$-$C_8$-Alkyl, $C_5$-$C_8$-Cycloalkyl oder $C_7$-$C_9$-Phenylalkyl und $R^7$ $C_1$-$C_8$-Alkyl, $C_5$-$C_8$-Cycloalkyl oder Phenyl ist, oder

b) eine Gruppe der Formel III sind, worin m 1, 2 oder 3 ist,

Y —O— oder $—N(R^9)—$ ist, worin $R^9$ Wasserstoff, $C_1$-$C_{12}$-Alkyl oder Cyclohexyl bedeutet oder gleich D ist,

D eine Gruppe der Formel V ist, in der R Wasserstoff bedeutet und $R^{10}$ $C_1$-$C_6$-Alkyl, Benzyl, durch —CN oder $—COOR^{15}$ substituiertes $C_1$-$C_4$-Alkyl, eine Gruppe $—CO—R^{18}$, $—COOR^{15}$, $—CON(R^{16})(R^{17})$, $—CH_2—CH(R^{19})—OR^{20}$, $—SOR^{21}$, $—SO_2R^{21}$, $—OR^{15}$ oder $—OOC—R^{18}$ bedeutet, worin $R^{15}$ $C_1$-$C_8$-Alkyl ist, $R^{16}$ $C_1$-$C_8$-Alkyl, Cyclohexyl oder Phenyl und $R^{17}$ Wasserstoff oder $C_1$-$C_8$-Alkyl bedeuten oder $R^{16}$ und $R^{17}$ zusammen mit dem N-Atom Pyrrolidin, Piperidin oder Morpholin bedeuten, $R^{18}$ $C_1$-$C_4$-Alkyl, $C_2$-$C_3$-Alkenyl, Cyclohexyl, Phenyl oder Benzyl ist, $R^{19}$ Wasserstoff oder Methyl ist, $R^{20}$ Wasserstoff, $C_1$-$C_8$-Alkyl oder eine Gruppe der Formel IX

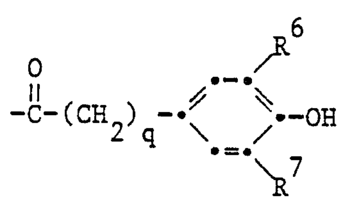
(IX)

mit q = 0, 1 oder 2 bedeutet und $R^{21}$ $C_1$-$C_4$-Alkyl, Phenyl oder p-Tolyl bedeutet, oder c) Wasserstoff sind,

$R^4$ und $R^5$ unabhängig voneinander Wasserstoff oder $C_1$-$C_{12}$-Alkyl sind oder eine der unter a) oder

b) genannten Bedeutungen haben und von den Resten $R^1$, $R^2$, $R^3$, $R^4$ und $R^5$ mindestens einer eine sterisch gehinderte Phenolgruppe enthält und mindestens einer eine N-substituierte 2,2,6,6-Tetramethylpiperidingruppe enthält.

Verbindungen der Formel I, worin X eine Gruppe —N($R^3$)— ist, stellen Isocyanursäureester dar. Unter dieser Gruppe von Stabilisatoren sind solche bevorzugt, bei denen

$R^1$ und $R^2$ eine Gruppe der Formel IIa sind, worin $R^6$ Wasserstoff oder $C_1$-$C_4$-Alkyl und $R^7$ $C_1$-$C_4$-Alkyl bedeuten,

$R^3$ eine Gruppe der Formel III ist, worin m 1 oder 2 bedeutet,

Y —O— oder —N($R^9$)— ist, worin $R^9$ Wasserstoff oder $C_1$-$C_{12}$-Alkyl bedeutet oder gleich D ist, und

D eine Gruppe der Formel V ist, in der R Wasserstoff bedeutet und

$R^{10}$ Methyl, Allyl, Benzyl, Acetyl, Acryloyl, Methoxy, Aethoxy oder —$CH_2CH_2OR^{20}$ bedeutet, wobei $R^{20}$ Wasserstoff oder eine Gruppe der Formel IX

bedeutet, worin q = 0, 1 oder 2 ist und $R^6$ und $R^7$ die oben gegebene Bedeutung haben.

Verbindungen der Formel I, worin X eine Gruppe —C($R^4$)($R^5$)— ist, stellen Barbitursäurederivate dar. Unter dieser Gruppe von Stabilisatoren sind solche bevorzugt, bei denen

$R^1$ und $R^2$ eine Gruppe der Formel IIa sind, worin $R^6$ Wasserstoff oder $C_1$-$C_4$-Alkyl und $R^7$ $C_1$-$C_4$-Alkyl bedeuten,

$R^4$ eine Gruppe der Formel III ist, worin m 1 oder 2 bedeutet,

Y —O— oder —N($R^9$)— ist, worin $R^9$ Wasserstoff oder $C_1$-$C_{12}$-Alkyl bedeutet oder gleich D ist und

D eine Gruppe der Formel V ist, in der R Wasserstoff bedeutet und $R^{10}$ Methyl, Allyl, Benzyl, Acetyl, Acryloyl, Methoxy, Acetoxy oder —$CH_2CH_2OR^{20}$ bedeutet, worin $R^{20}$ Wasserstoff oder eine Gruppe der Formel IX ist,

worin q = 0, 1 oder 2 ist, $R^6$ Wasserstoff oder $C_1$-$C_4$-Alkyl und $R^7$ $C_1$-$C_4$-Alkyl bedeuten und

$R^5$ dieselbe Bedeutung wie $R^4$ hat oder $C_1$-$C_{12}$-Alkyl ist, oder dieselbe Bedeutung wie $R^1$ hat, insbesondere aber solche, bei denen

$R^1$ und $R^2$ eine Gruppe der Formel III sind, worin m 1 oder 2 bedeutet, Y —O— oder —N($R^9$)— ist, worin $R^9$ Wasserstoff oder $C_1$-$C_{12}$-Alkyl bedeutet oder gleich D ist und D eine Gruppe der Formel V ist, in der R Wasserstoff bedeutet und $R^{10}$ Methyl, Allyl, Benzyl, Acetyl, Acryloyl, Metoxy, Acetoxy oder —$CH_2CH_2OR^{20}$ bedeutet, worin $R^{20}$ Wasserstoff oder eine Gruppe der Formel IX ist,

worin q = 0, 1 oder 2 ist, $R^6$ Wasserstoff oder $C_1$-$C_4$-Alkyl und $R^7$ $C_1$-$C_4$-Alkyl bedeuten,

$R^4$ eine Gruppe der Formel IIa ist, worin $R^6$ und $R^7$ die oben gegebenen Bedeutung haben und

$R^5$ dieselbe Bedeutung wie $R^4$ hat oder $C_1$-$C_{12}$-Alkyl ist oder dieselbe Bedeutung wie $R^1$ hat.

Ein Teil der hier beschriebenen Verbindungen der Formel I, worin X eine Gruppe —N($R^3$)— ist, ist aus der DE-OS 27 30 397 oder der US-PS 4 317 911 bekannt, wo ihre Herstellung sowie ihre Verwendung als Stabilisatoren für Kunststoffe ausführlich beschrieben ist.

Ein Teil der hier beschriebenen Verbindungen der Formel I, worin X eine Gruppe —C($R^4$)($R^5$)— ist, ist aus der DE-OS 27 30 503 oder der US-PS 4 185 007 bekannt, wo ebenfalls ihre Herstellung und ihre Verwendung in Kunststoffen beschrieben ist.

Soweit die hier beschriebenen Verbindungen neu sind, können sie in Analogie zu den bekannten Verbindungen hergestellt werden.

Beispiele für einzelne Verbindungen der Formel I, worin X eine Gruppe —N($R^3$)— ist, sind die Verbindungen der folgenden Formeln :

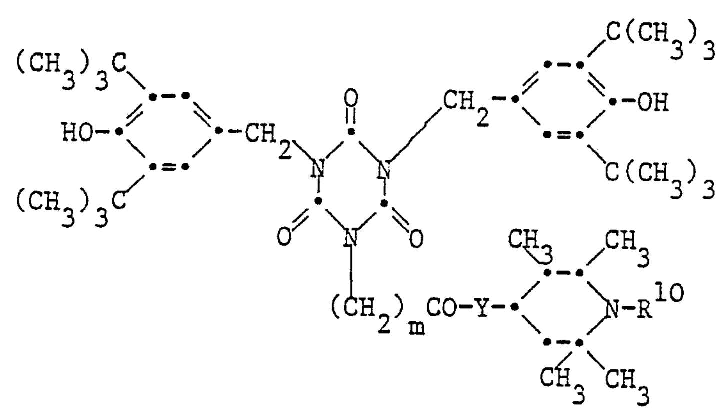

| Verbindung Nr. | m | Y | $R^{10}$ |
|---|---|---|---|
| 1 | 1 | -O- | $-CO-CH=CH_2$ |
| 2 | 1 | -O- | $-CH_3$ |

(Fortsetzung)

| Verbindung Nr. | m | Y | $R^{10}$ |
|---|---|---|---|
| 3 | 1 | -O- | $-CH_2-$ 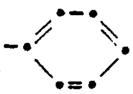 |
| 4 | 1 | -O- | $-CO-N(C_2H_5)_2$ |
| 5 | 1 | -NH- | $-CO-CH=CH_2$ |
| 6 | 1 | -NH- | $-CO-CH_3$ |
| 7 | 1 | $-N(C_4H_9)-$ | $-CO-CH_3$ |
| 8 | 2 | -O- | $-SO_2-$ 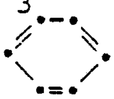 $-CH_3$ |
| 9 | 2 | -NH- | $-CH_2CN$ |

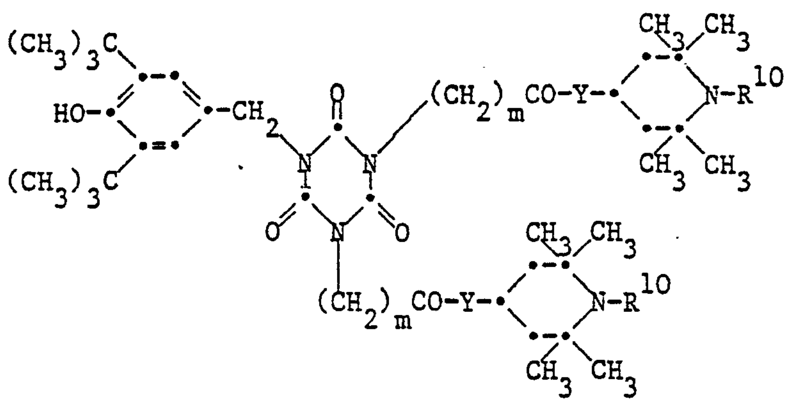

| | | | |
|---|---|---|---|
| Nr. 10 | m=1 | Y = -O- | $R^{10} = -CO-CH=CH_2$ |
| 11 | 1 | -O- | $-CO-N(C_2H_5)_2$ |
| 12 | 1 | -O- | $-CH_2-$ 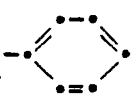 |
| 13 | 1 | -O- | $-CH_3$ |
| 14 | 1 | -O- | $-CO-CH_3$ |
| 15 | 1 | -NH- | $-CH_3$ |
| 16 | 2 | -O- | $-CO-CH=CH_2$ |
| 17 | 2 | -O- | $-OOC-CH_3$ |
| 18 | 2 | $-N(C_3H_7)-$ | $-CO-CH_3$ |

(Fortsetzung)

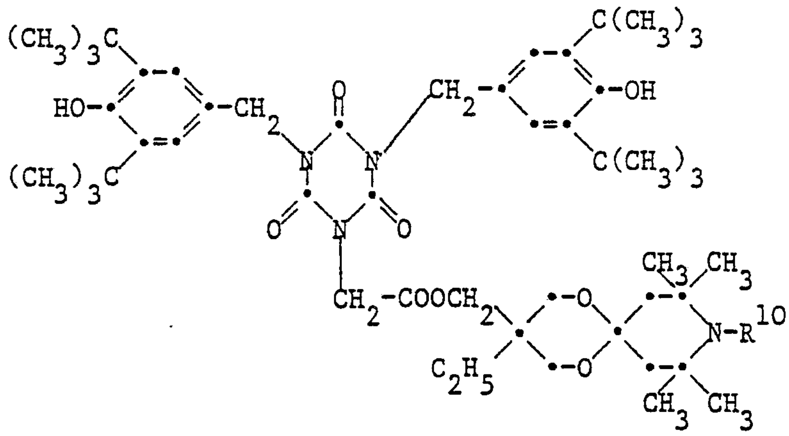

Nr. 19 $R^{10}$ = $-CH_3$

20 $-CO-CH=CH_2$

21 $-CH_2CH_2OOCCH_3$

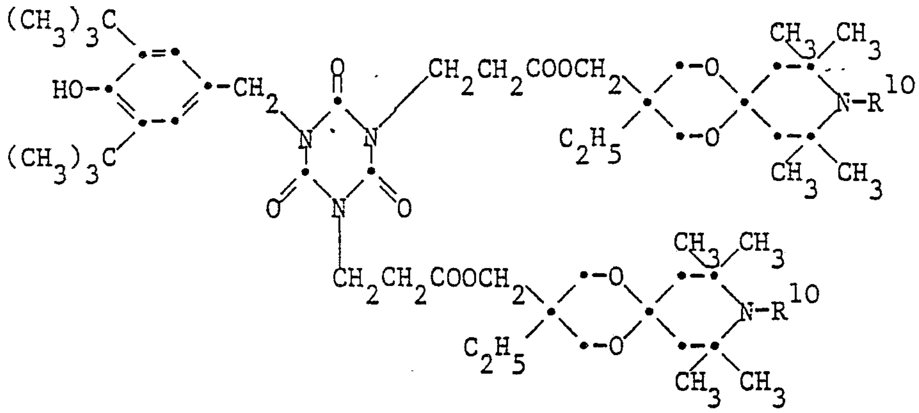

Nr. 22 $R^{10}$ = $-COCH_3$

23 $-CH_2CH=CH_2$

---

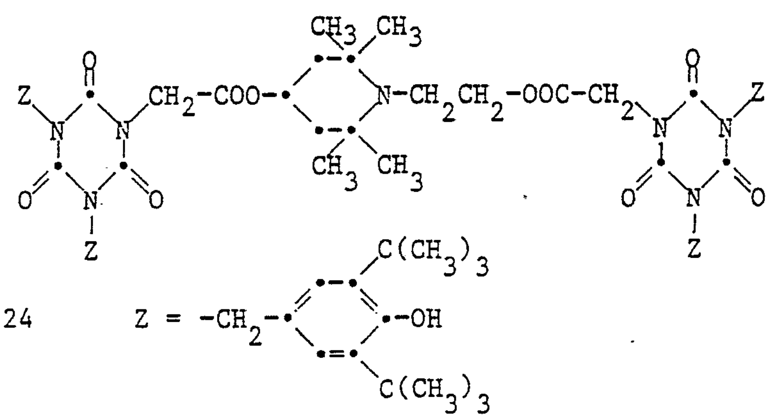

Nr. 24 Z =

Beispiele für Verbindungen der Formel I, worin X eine Gruppe —C($R^4$)($R^5$)— ist, sind die Verbindungen der folgenden Formeln :

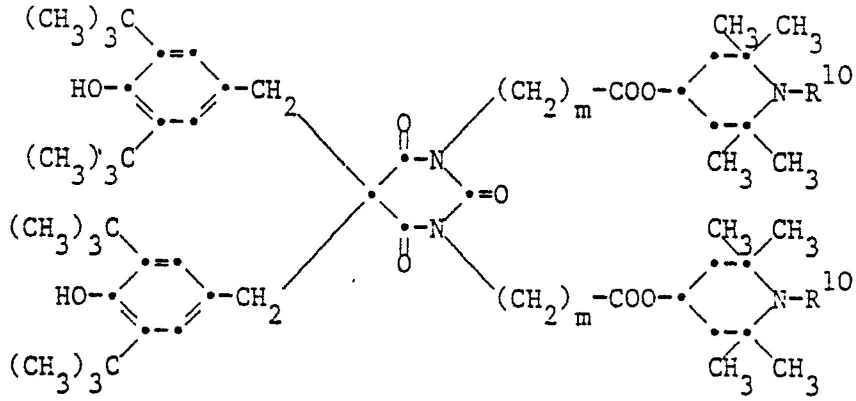

| Nr. | m | $R^{10}$ |
|---|---|---|
| 25 | m = 1 | $R^{10}$ = $-CH_3$ |
| 26 | 1 | $-CO-CH=CH_2$ |
| 27 | 2 | $-CH_2CH=CH_2$ |
| 28 | 1 | $-CH_2-$ 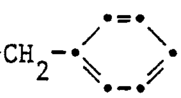 |
| 29 | 2 | $-CH_3$ |

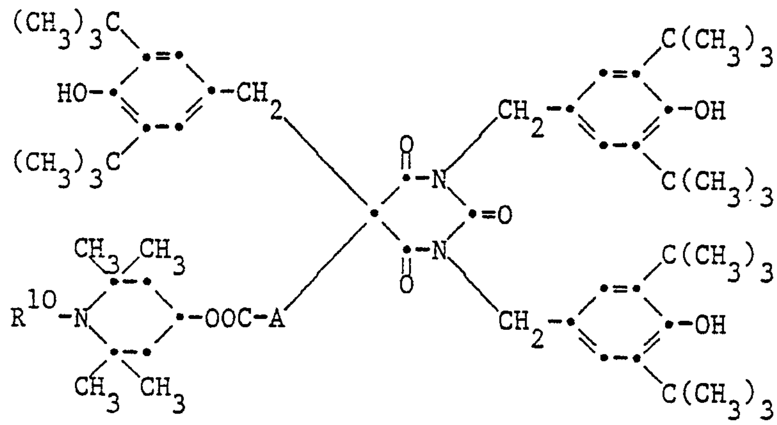

| Nr. | A | $R^{10}$ |
|---|---|---|
| 30 | A = $-CH_2-$ | $R^{10}$ = $-CO-CH=CH_2$ |
| 31 | $-CH_2CH_2-$ | $-CH_3$ |
| 32 | $-CH(CH_3)-CH_2-$ | $-COCH_3$ |
| 33 | $-CH(CH_3)-CH_2-$ | $-CH_3$ |

(Fortsetzung)

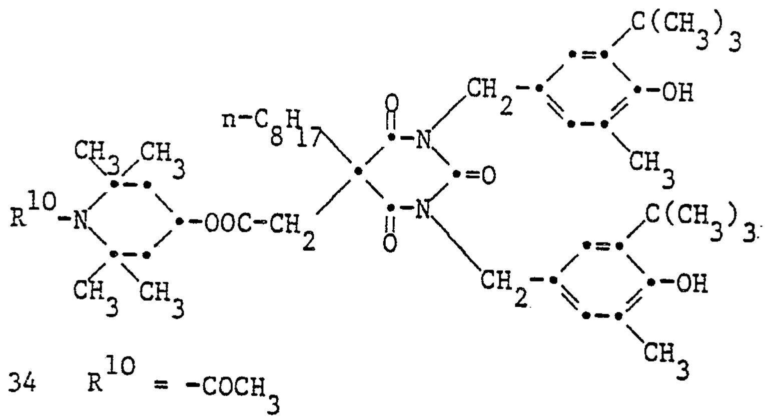

Nr. 34 $R^{10}$ = $-COCH_3$

35 $-CH_3$

Nr. 36

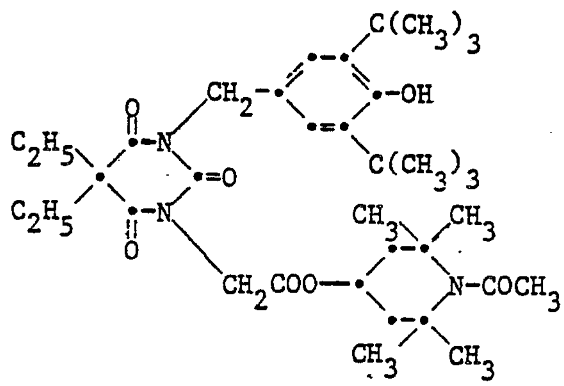

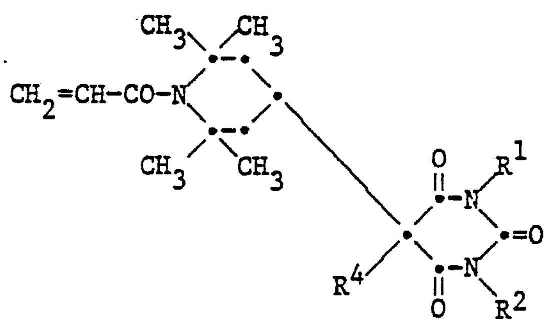

Nr. 37 $R^1$ = $R^2$ = $R^4$ =

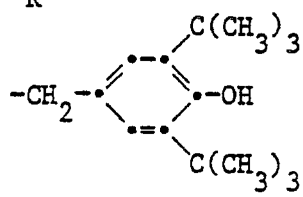

Nr. 38

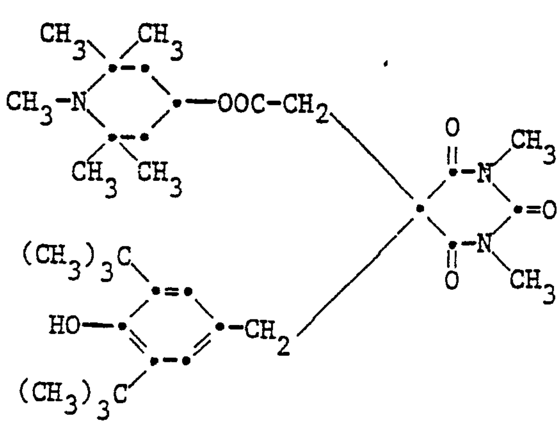

Die Stabilisatoren der Formel I können allein oder zusammen mit anderen Verbindungen in bekannter Weise in ein photographisches Material eingearbeitet werden.

In der Regel werden die Stabilisatoren allein oder zusammen mit anderen Verbindungen, insbesondere mit den Farbkupplern, in Form einer Dispersion in das photographische Material eingearbeitet, wobei diese Dispersion entweder kein Lösungsmittel oder hoch- oder tiefsiedende Lösungsmittel oder ein Gemisch solcher Lösungsmittel enthält. Eine weitere geeignete Einarbeitungsform besteht darin, dass man die Stabilisatoren allein oder zusammen mit weiteren Verbindungen zusammen mit einem Polymer in form eines Latex in das photographische Material einarbeitet.

Die Dispersionen werden dann zur Herstellung der Schichten von farbphotographischen Aufzeichnungsmaterialien verwendet. Diese Schichten können z. B. Zwischen- oder Schutzschichten, insbesondere jedoch lichtempfindliche (blau-, grün und rot-empfindliche) Silberhalogenidemulsionsschichten sein, in denen bei der Entwicklung des belichteten Aufzeichnungsmaterials aus den entsprechenden Farbkupplern die Blaugrün (Cyan)-, Purpur (Magenta)- und Gelbfarbstoffe gebildet werden.

Die Silberhalogenidschichten können beliebige Farbkuppler, insbesondere Blaugrün-, Purpur- und Gelb-Kuppler, die zur Bildung der genannten Farbstoffe und damit der Farbbilder verwendet werden, enthalten.

Da das Substrat die Wirkung und Stabilität der Stabilisatoren beeinflusst, werden Substrate (Lösungsmittel, Polymere) bevorzugt, die zusammen mit den Stabilisatoren eine möglichst gute Beständigkeit der zu stabilisierenden Materialien ergeben.

In der Regel werden die Stabilisatoren in Schichten eingearbeitet, die zusätzlich eine nach üblichen Methoden hergestellte und sensibilisierte Silberhalogenid-Dispersion enthalten. Sie können jedoch auch in zu Silberhalogenid enthaltenden Schichten benachbarten Schichten vorhanden sein.

Die erfindungsgemässen photographischen Materialien besitzen einen üblichen Aufbau und Komponenten, die die Wirksamkeit der Stabilisatoren verstärken oder zumindest nicht nachteilig beeinflussen.

Im photographischen Aufzeichnungsmaterial gemäss vorliegender Erfindung können die Stabilisatoren der Formel I ausser mit den Farbkupplern zusätzlich auch mit Ultraviolettabsorbern oder anderen Lichtschutzmitteln in der gleichen Schicht kombiniert werden.

Wenn die Diffusionstransfermethode angewendet wird, kann der Stabilisator auch in eine Empfangsschicht eingearbeitet werden.

Die erfindungsgemässen farbphotographischen Materialien können in bekannter Weise verarbeitet werden. Ferner können sie im Verlauf oder nach der Verarbeitung in einer Weise behandelt werden, die ihre Stabilität weiter erhöht, beispielsweise durch die Behandlung in einem Stabilisatorbad oder das Aufbringen eines Schutzüberzuges.

Die erfindungsgemäss einzusetzenden Stabilisatoren eignen sich in gewissen Fällen auch zum Schutz farbphotographischer Schichten in denen die Farbstoffe direkt in die Emulsion eingelagert werden und das Bild durch selektive Bleichung erzeugt wird.

Die Menge des Stabilisators oder der Stabilisatoren kann in weiten Grenzen schwanken und liegt etwa im Bereich von 1 bis 2 000 mg, vorzugsweise 100 bis 800 und insbesondere 200-500 mg pro $m^2$ der Schicht, in die es (sie) eingearbeitet wird (werden).

Falls das photographische Material einen oder mehrere UV-Absorber enthält, so kann dieser mit dem Stabilisator zusammen in einer Schicht oder auch in einer benachbarten Schicht vorhanden sein. Die Menge an UV-Absorber kann in weiten Grenzen schwanken und liegt etwa im Bereich von 200-2 000 mg, vorzugsweise 400-1 000 mg pro $m^2$ der Schicht.

Beispiele für geeignete UV-Absorber sind solche vom Benzophenon-, Acrylnitril-, Thiazolidon-, Benztriazol-, Oxazol-, Thiazol- und Imidazoltyp.

Die mit dem erfindungsgemässen Aufzeichnungsmaterial durch Belichtung und Entwicklung erhaltenen Farbbilder zeigen eine sehr gute Lichtechtheit gegenüber sichtbarem und ultraviolettem Licht. Die Verbindungen der Formel I sind praktisch farblos, so dass es zu keiner Verfärbung der Bilder kommt ; ausserdem sind sie gut verträglich mit den üblichen, in den einzelnen Schichten vorhandenen photographischen Zusatzstoffen. Aufgrund ihrer guten Wirksamkeit kann man ihre Einsatzmenge herabsetzen und vermeidet so ihre Ausfällung oder ihr Auskristallisieren, wenn man sie als organische Lösung in die wässerigen Bindemittelemulsionen, die für die Herstellung photographischer Schichten verwendet werden, einarbeitet. Die einzelnen nach der Belichtung des photographischen Aufzeichnungsmaterials notwendigen Verarbeitungsschritte zur Herstellung der Farbbilder werden durch die Stabilisatoren der Formel I nicht nachteilig beeinflusst. Ferner kann die bei blauempfindlichen Emulsionen häufig auftretende sogenannte Druckschleierbildung weitgehend zurückgedrängt werden. Diese kann z. B. auftreten, wenn auf photographische Materialien (Silberhalogenidemulsionsschichten, die auf einem Träger aus natürlichen oder synthetischen Materialien befinden) mechanische Beanspruchungen, z. B. Drehen, Biegen oder Reiben, während der Herstellung oder während der Behandlung vor der Entwicklung ausgeübt werden. (T.H. James, The Theory of Photographic Process 4. Auflage, Macmillan, New York, N.Y. 1977, Seite 23 ff., S. 166 ff.).

## Beispiel

0,087 g des Gelbkupplers der Formel

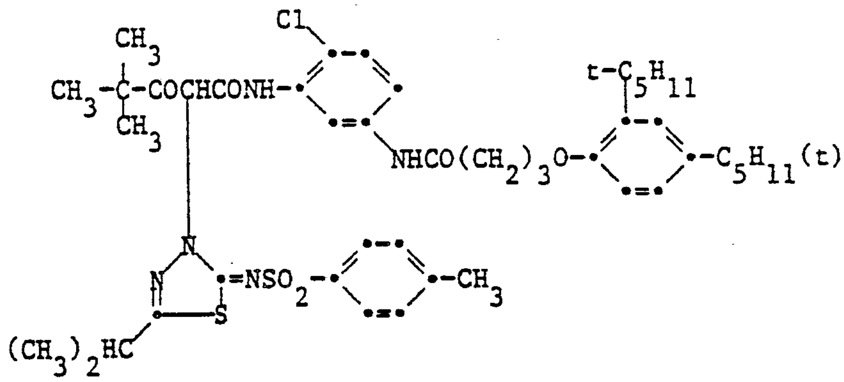

und 0,026 g eines der in der nachfolgenden Tabelle angegebenen Stabilisatoren werden in 2,0 ml eines Gemisches von Trikresylphosphat/Ethylacetat (1,5 g in 100 ml) gelöst. Zu dieser Lösung gibt man 7,0 ml einer 6 %igen Gelatinelösung, 0,5 ml einer 8 %igen Lösung des Netzmittels der Formel

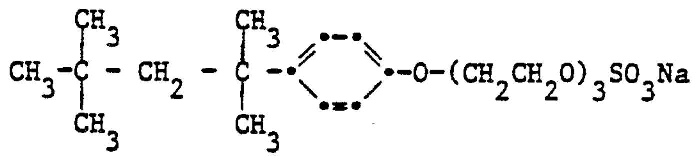

in Isopropanol/Wasser (3 : 4) und 0,5 ml Wasser und emulgiert mit Ultraschall bei einer Leistung von 100 Watt während 5 Minuten.

Zu 2,5 ml der so erhaltenen Emulsion gibt man 2,0 ml einer Silberbromid-Emulsion mit einem Silbergehalt von 6,0 g pro Liter, 0,7 ml einer 1 %igen wässerigen Lösung des Härters der Formel

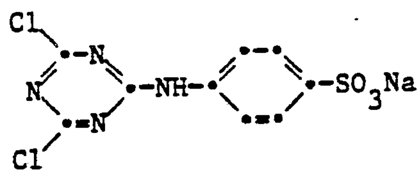

und 3,8 ml Wasser, stellt das Gemisch auf einen pH-Wert von 6,5 und vergiesst es auf ein auf eine Glasplatte aufgezogenes substriertes, kunststoffbeschichtetes, weisses Papier.

Nach dem Erstarren wird in einem Trockenschrank mit Umluft bei Raumtemperatur getrocknet.

Nach 7 Tagen werden auf 35 × 180 mm geschnittene Proben hinter einem Stufenkeil mit 3 000 Lux · s belichtet und anschliessend im Ektaprint® 2-Prozess der Firma Kodak verarbeitet.

Die so erhaltenen Gelbkeile werden in einem Atlas Weather-Ometer mit einer 2 500 W-Xenonlampe mit total 42 KJoule/$cm^2$ bestrahlt (eine Vergleichsprobe enthält kein Lichtschutzmittel). Der dabei eingetretene Farbdichteverlust wird bestimmt durch Messung der Farbdichte bei $\lambda_{max}$ mit einem Densitometer (TR 924 A der Fa. Macbeth).

Die Ergebnisse sind in der nachfolgenden Tabelle aufgeführt.

| Stabilisator Verbindung Nr. | Prozentualer Farbdichteverlust |
|---|---|
| 1 | 20 |
| 3 | 22 |
| 4 | 21 |
| 5 | 22 |
| 12 | 20 |
| 13 | 16 |
| 14 | 20 |
| 20 | 22 |
| ohne Stab. | 36 |

**Patentansprüche**

1. Farbphotographisches Aufzeichnungsmaterial, das in mindestens einer lichtempfindlichen Silberhalogenid-Emulsionsschicht, einer Zwischenschicht, einer Bildempfangsschicht und/oder einer Schutzschicht als Stabilisator mindestens eine Verbindung der Formel I enthält,

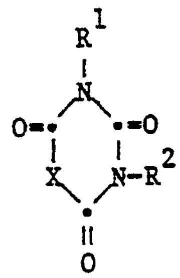

(I)

worin X eine Gruppe

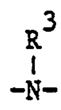 oder 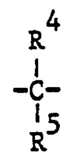

darstellt und $R^1$, $R^2$, $R^3$, $R^4$ und $R^5$ einwertige organische Reste sind, von denen mindestens einer eine sterisch gehinderte Phenolgruppe und mindestens einer eine Polyalkylpiperidingruppe enthält.

2. Farbphotographisches Aufzeichnungsmaterial gemäss Anspruch 1, enthaltend mindestens eine Verbindung der Formel I, worin X eine Gruppe $-N(R^3)-$ oder $-C(R^4)(R^5)-$ darstellt, $R^1$, $R^2$ und $R^3$ unabhängig voneinander entweder

a) eine Gruppe der Formel II sind

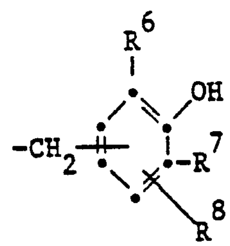

(II)

worin

$R^6$ Wasserstoff, $C_1$-$C_8$-Alkyl, $C_5$-$C_8$-Cycloalkyl, $C_7$-$C_9$-Phenylalkyl, Phenyl oder $C_7$-$C_{10}$-Alkylphenyl bedeutet,

$R^7$ $C_1$-$C_8$-Alkyl, $C_5$-$C_8$-Cycloalkyl, $C_7$-$C_9$-Phenylalkyl, Phenyl oder $C_7$-$C_{10}$-Alkylphenyl bedeutet,

$R^8$ Wasserstoff oder Methyl ist,

oder

b) eine Gruppe der Formel III oder IV sind,

$$-C_mH_{2m}-CO-Y-D \quad \text{(III)}$$

$$-C_mH_{2m}-CO-O-C_nH_{2n}-E \quad \text{(IV)}$$

worin

m 1-4 und

n 2-5 ist,

Y $-O-$ oder $-NR^9-$ ist und $R^9$ Wasserstoff, $C_1$-$C_{12}$-Alkyl, $C_5$-$C_{12}$-Cycloalkyl, $C_3$-$C_{12}$-Alkoxyalkyl, $C_4$-$C_{12}$-Dialkylaminoalkyl oder eine Gruppe der Formel

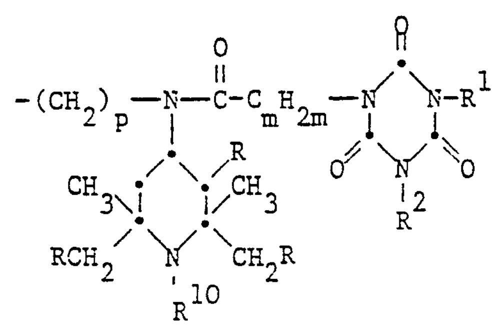

bedeutet, worin p 2-12 ist, oder $R^9$ gleich D ist,
D eine Gruppe der Formel V, VI oder VII ist,

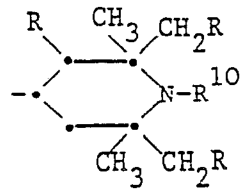

(V)

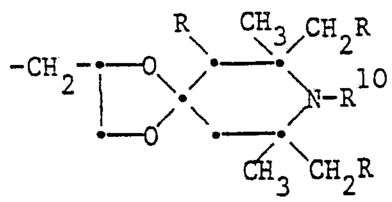

(VI)

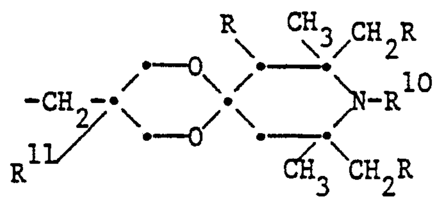

(VII)

und E eine Gruppe der Formel VIII ist,

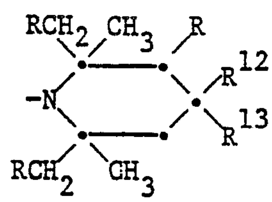

(VIII)

worin

R Wasserstoff oder Methyl ist,

$R^{10}$ Hydroxyl, $C_1$-$C_{12}$-Alkyl, $C_3$-$C_6$-Alkenyl, $C_3$-$C_4$-Alkinyl, $C_7$-$C_{12}$-Phenylalkyl, Glycidyl, durch Halogen, —CN, —$COOR^{15}$ oder —$CON(R^{16})(R^{17})$ substituiertes $C_1$-$C_4$-Alkyl, eine Gruppe —CO—$R^{18}$, —CO—$OR^{15}$, —CO—$N(R^{16})(R^{17})$, —$CH_2$—$CH(R^{19})$—$OR^{20}$, —SO—$R^{21}$, —$SO_2$—$R^{21}$, —$OR^{15}$ oder —OOC—$R^{18}$ bedeutet,

$R^{11}$ Methyl oder Ethyl ist,

$R^{12}$ Wasserstoff, —$OR^{22}$, —OOC—$R^{18}$, oder —$N(R^9)$—CO—$R^{18}$ und

$R^{13}$ Wasserstoff, —CN, —$COOR^{15}$ oder —$CONH_2$ bedeuten oder

$R^{12}$ und $R^{13}$ zusammen eine Gruppe der folgenden Formeln bilden,

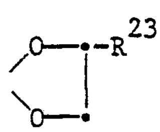

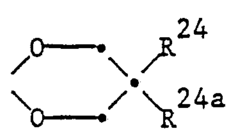

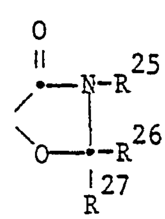

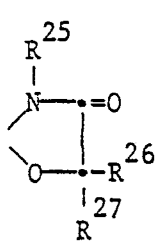

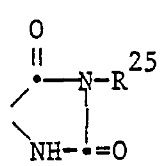

$R^{15}$ $C_1$-$C_{12}$-Alkyl, Allyl, Benzyl oder Cyclohexyl bedeutet,
$R^{16}$ $C_1$-$C_{12}$-Alkyl, Allyl, Cyclohexyl, Benzyl oder Phenyl, und
$R^{17}$ Wasserstoff oder $C_1$-$C_8$-Alkyl bedeuten oder
$R^{16}$ und $R^{17}$ zusammen mit dem N-Atom einen 5- oder 6-gliedrigen heterocyclischen Ring bilden,
$R^{18}$ Wasserstoff, $C_1$-$C_{12}$-Alkyl, $C_2$-$C_6$-Alkenyl, Chlormethyl, $C_5$-$C_{12}$-Cycloalkyl, $C_7$-$C_{12}$-Phenylalkyl, Phenyl, $C_7$-$C_{10}$-Alkylphenyl oder durch 1 oder 2 $C_1$-$C_4$-Alkylgruppe und eine Hydroxygruppe substituiertes Phenyl, Phenylmethyl oder Phenylethyl bedeutet,
$R^{19}$ Wasserstoff, $C_1$-$C_4$-Alkyl, $C_2$-$C_{13}$-Alkoxymethyl, Phenyl oder Phenoxymethyl bedeutet,
$R^{20}$ Wasserstoff, $C_1$-$C_{12}$-Alkyl, —CO—$R^{18}$ oder —CO—N($R^{16}$)($R^{17}$) bedeutet,
$R^{21}$ $C_1$-$C_{12}$-Alkyl, Phenyl oder $C_7$-$C_{18}$-Alkylaryl bedeutet,
$R^{22}$ Wasserstoff, $C_1$-$C_{12}$-Alkyl, Allyl oder Benzyl ist,
$R^{23}$ Wasserstoff, Methyl oder Ethyl ist,
$R^{24}$ und $R^{24a}$ unabhängig voneinander H oder $C_1$-$C_4$-Alkyl bedeuten,
$R^{25}$ Wasserstoff, $C_1$-$C_{12}$-Alkyl, $C_3$-$C_6$-Alkenyl oder $C_7$-$C_{12}$-Phenylalkyl bedeutet,
$R^{26}$ Wasserstoff, $C_1$-$C_{12}$-Alkyl, $C_3$-$C_8$-Cycloalkyl oder Benzyl und
$R^{27}$ $C_1$-$C_{12}$-Alkyl, $C_5$-$C_8$-Cycloalkyl oder Phenyl ist, oder
$R^{26}$ und $R^{27}$ zusammen mit dem C-Atom, an das sie gebunden sind, einen $C_5$-$C_{12}$-Cycloalkan- oder Alkylcycloalkanring bilden,
oder

c) Wasserstoff, $C_1$-$C_{18}$-Alkyl, $C_3$-$C_6$-Alkenyl, $C_7$-$C_{12}$-Phenylalkyl, $C_3$-$C_{12}$-Alkoxyalkyl oder $C_3$-$C_{14}$-Alkoxycarbonylalkyl darstellen,
$R^4$ Wasserstoff, $C_1$-$C_{18}$-Alkyl, $C_5$-$C_8$-Cycloalkyl, eine Gruppe der Formel II, eine Gruppe der Formel III oder IV oder eine Gruppe —$C_mH_{2m}$—COO($C_1$-$C_4$-Alkyl) bedeutet und
$R^5$ Wasserstoff, $C_1$-$C_{18}$-Alkyl, $C_5$-$C_8$-Cycloalkyl, Phenyl, $C_7$-$C_9$-Phenylalkyl, eine Gruppe der Formel II, eine Gruppe der Formel III oder IV oder eine Gruppe der Formel V bedeutet,
wobei von den Resten $R^1$, $R^2$, $R^3$, $R^4$, $R^5$ mindestens einer eine sterisch gehinderte Phenolgruppe enthält und mindestens einer eine Polyalkylpiperidingruppe enthält.

3. Farbphotographisches Aufzeichnungsmaterial gemäss Anspruch 1, enthaltend mindestens eine Verbindung der Formel I, worin X eine Gruppe —N($R^3$)— oder —C($R^4$)($R^5$)— darstellt und von den Resten $R^1$, $R^2$, $R^3$, $R^4$ und $R^5$ mindestens einer eine sterisch gehinderte Phenolgruppe und mindestens einer eine N-substituierte 2,2,6,6-Tetramethylpiperidingruppe enthält.

4. Farbphotographisches Aufzeichnungsmaterial gemäss Anspruch 2, enthaltend eine Verbindung der Formel I, worin X eine Gruppe —N($R^3$)— oder —C($R^4$)($R^5$)— darstellt und $R^1$, $R^2$ und $R^3$ unabhängig voneinander entweder

a) eine Gruppe der Formel IIa oder IIb ist,

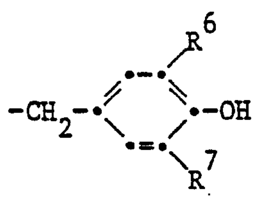
(IIa)

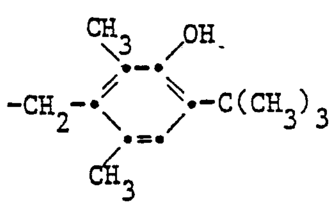
(IIb)

worin
$R^6$ Wasserstoff, $C_1$-$C_8$-Alkyl, $C_5$-$C_8$-Cycloalkyl oder $C_7$-$C_9$-Phenylalkyl und
$R^7$ $C_1$-$C_8$-Alkyl, $C_5$-$C_8$-Cycloalkyl oder Phenyl ist,
oder

b) eine Gruppe der Formel III sind, worin
m 1, 2 oder 3 ist,
Y —O— oder —N($R^9$)— ist, worin $R^9$ Wasserstoff, $C_1$-$C_{12}$-Alkyl oder Cyclohexyl bedeutet oder gleich D ist,
D eine Gruppe der Formel V ist, in der R Wasserstoff bedeutet und $R^{10}$ $C_1$-$C_6$-Alkyl, Allyl, Benzyl, durch —CN oder —COOR$^{15}$ substituiertes $C_1$-$C_4$-Alkyl, eine Gruppe —CO—$R^{18}$, —COOR$^{15}$, —CON($R^{16}$)($R^{17}$), —$CH_2$—CH($R^{19}$)—OR$^{20}$, —SOR$^{21}$, —$SO_2R^{21}$, —OR$^{15}$ oder —OOC—$R^{18}$ bedeutet.

worin $R^{15}$ $C_1$-$C_8$-Alkyl ist, $R^{16}$ $C_1$-$C_8$-Alkyl, Cyclohexyl oder Phenyl und $R^{17}$ Wasserstoff oder $C_1$-$C_8$-Alkyl bedeuten oder $R^{16}$ und $R^{17}$ zusammen mit dem N-Atom Pyrrolidin, Piperidin oder Morpholin bedeuten, $R^{18}$ $C_1$-$C_4$-Alkyl, $C_2$-$C_3$-Alkenyl, Cyclohexyl, Phenyl oder Benzyl ist, $R^{19}$ Wasserstoff oder Methyl ist, $R^{20}$ Wasserstoff, $C_1$-$C_8$-Alkyl oder eine Gruppe der Formel IX

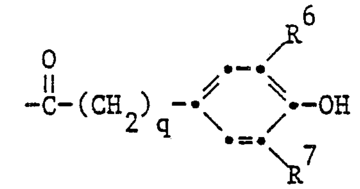

(IX)

mit q = 0, 1 oder 2 bedeutet und $R^{21}$ $C_1$-$C_4$-Alkyl, Phenyl oder p-Tolyl bedeutet, oder

c) Wasserstoff sind,

$R^4$ und $R^5$ unabhängig voneinander Wasserstoff oder $C_1$-$C_{12}$-Alkyl sind oder eine der unter a) oder b) genannten Bedeutungen haben und von den Resten $R^1$, $R^2$, $R^3$, $R^4$ und $R^5$ mindestens einer eine sterisch gehinderte Phenolgruppe enthält und mindestens einer eine N-substituierte 2,2,6,6-Tetramethylpiperidingruppe enthält.

5. Farbphotographisches Aufzeichnungsmaterial gemäss Anspruch 4, enthaltend eine Verbindung der Formel I, worin

X eine Gruppe —N($R^3$)— ist,

$R^1$ und $R^2$ eine Gruppe der Formel IIa sind, worin $R^6$ Wasserstoff oder $C_1$-$C_4$-Alkyl und $R^7$ $C_1$-$C_4$-Alkyl bedeuten,

$R^3$ eine Gruppe der Formel III ist, worin m 1 oder 2 bedeutet,

Y —O— oder —N($R^9$)— ist, worin $R^9$ Wasserstoff oder $C_1$-$C_{12}$-Alkyl bedeutet oder gleich D ist, und

D eine Gruppe der Formel V ist, in der R Wasserstoff bedeutet und $R^{10}$ Methyl, Allyl, Benzyl, Acetyl, Acryloyl, Methoxy, Aethoxy oder —$CH_2CH_2OR^{20}$ bedeutet, wobei $R^{20}$ Wasserstoff oder eine Gruppe der Formel IX

bedeutet, worin q = 0, 1 oder 2 ist und $R^6$ und $R^7$ die oben gegebene Bedeutung haben.

6. Farbphotographisches Aufzeichnungsmaterial gemäss Anspruch 4, enthaltend eine Verbindung der Formel I, worin

X eine Gruppe —C($R^4$)($R^5$)— ist,

$R^1$ und $R^2$ eine Gruppe der Formel IIa sind, worin $R^6$ Wasserstoff oder $C_1$-$C_4$-Alkyl und $R^7$ $C_1$-$C_4$-Alkyl bedeuten,

$R^4$ eine Gruppe der Formel III ist, worin m 1 oder 2 bedeutet,

Y —O— oder —N($R^9$)— ist, worin $R^9$ Wasserstoff oder $C_1$-$C_{12}$-Alkyl bedeutet oder gleich D ist und

D eine Gruppe der Formel V ist, in der R Wasserstoff bedeutet und $R^{10}$ Methyl, Allyl, Benzyl, Acetyl, Acryloyl, Methoxy, Acetoxy oder —$CH_2CH_2OR^{20}$ bedeutet, worin $R^{20}$ Wasserstoff oder eine Gruppe der Formel IX ist,

worin q = 0, 1 oder 2 ist, $R^6$ Wasserstoff oder $C_1$-$C_4$-Alkyl und $R^7$ $C_1$-$C_4$-Alkyl bedeuten und

$R^5$ dieselbe Bedeutung wie $R^4$ hat oder $C_1$-$C_{12}$-Alkyl ist, oder dieselbe Bedeutung wie $R^1$ hat.

7. Farbphotographisches Aufzeichnungsmaterial gemäss Anspruch 4, enthaltend eine Verbindung der Formel I, worin

X eine Gruppe —C($R^4$)($R^5$)— ist,

$R^1$ und $R^2$ eine Gruppe der Formel III sind, worin m 1 oder 2 bedeutet,

Y —O— oder —N($R^9$)— ist, worin $R^9$ Wasserstoff oder $C_1$-$C_{12}$-Alkyl bedeutet oder gleich D ist und

D eine Gruppe der Formel V ist, in der R Wasserstoff bedeutet und $R^{10}$ Methyl, Allyl, Benzyl, Acetyl, Acryloyl, Metoxy, Acetoxy oder —$CH_2CH_2OR^{20}$ bedeutet, worin $R^{20}$ Wasserstoff oder eine Gruppe der Formel IX ist,

worin q = 0, 1 oder 2 ist, $R^6$ Wasserstoff oder $C_1$-$C_4$-Alkyl und $R^7$ $C_1$-$C_4$-Alkyl bedeuten,

$R^4$ eine Gruppe der Formel IIa ist, worin $R^6$ und $R^7$ die oben gegebene Bedeutung haben und

$R^5$ dieselbe Bedeutung wie $R^4$ hat oder $C_1$-$C_{12}$-Alkyl ist oder dieselbe Bedeutung wie $R^1$ hat.

8. Farbphotographisches Aufzeichnungsmaterial gemäss Anspruch 1, dadurch gekennzeichnet, dass es neben einem Stabilisator der Formel I ein Lichtschutzmittel aus der Klasse der Ultraviolett-Absorber enthält.

9. Farbphotographisches Aufzeichnungsmaterial gemäss Anspruch 1, gekennzeichnet durch einen Gehalt an 1 bis 2 000, vorzugsweise 100 bis 800 mg pro $m^2$ an Verbindung der Formel I.

10. Verwendung der in Anspruch 1 definierten Verbindungen der Formel I als Stabilisatoren für farbphotographische Aufzeichnungsmaterialien.

**Claims**

1. A colour-photographic recording material which, in at least one light-sensitive silver halide

emulsion layer, one intermediate layer, one image-receiving layer and/or one protective layer, contains, as a stabiliser, at least one compound of the formula I

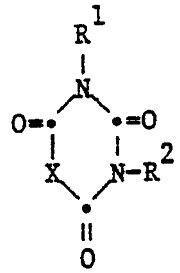

(I)

in which X is a group

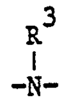

or

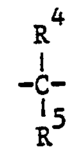

and $R^1$, $R^2$, $R^3$, $R^4$ and $R^5$ are monovalent organic radicals, at least one of which contains a sterically hindered phenol group and at least one of which contains a polyalkylpiperidine group.

2. A colour-photographic recording material according to claim 1, containing at least one compound of the formula I in which X is a group $—N(R^3)—$ or $—C(R^4)(R^5)—$, $R^1$, $R^2$ and $R^3$ independently of one another are either

a) a group of the formula II

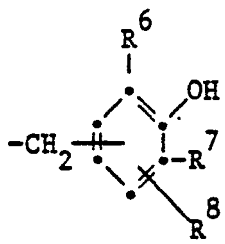

(II)

in which

$R^6$ is hydrogen, $C_1$-$C_8$-alkyl, $C_5$-$C_8$-cycloalkyl, $C_7$-$C_9$-phenylalkyl, phenyl or $C_7$-$C_{10}$-alkylphenyl,

$R^7$ is $C_1$-$C_8$-alkyl, $C_5$-$C_8$-cycloalkyl, $C_7$-$C_9$-phenylalkyl, phenyl or $C_7$-$C_{10}$-alkylphenyl, and

$R^8$ is hydrogen or methyl,

or

b) a group of the formula III or IV

$$—C_mH_{2m}—CO—Y—D \quad (III)$$

$$—C_mH_{2m}—CO—O—C_nH_{2n}—E \quad (IV)$$

in which

m is 1-4 and

n is 2-5,

Y is $—O—$ or $—NR^9—$ and $R^9$ is hydrogen, $C_1$-$C_{12}$-alkyl, $C_5$-$C_{12}$-cycloalkyl, $C_3$-$C_{12}$-alkoxyalkyl, $C_4$-$C_{12}$-dialkylaminoalkyl or a group of the formula

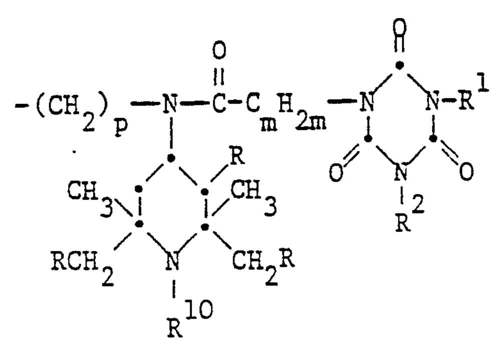

in which p is 2-12, or $R^9$ is D,

D is a group of the formula V, VI or VII

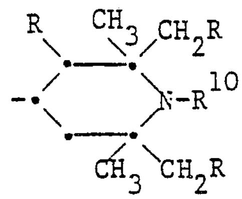

(V)

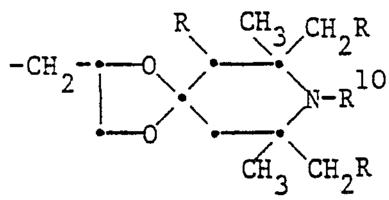

(VI)

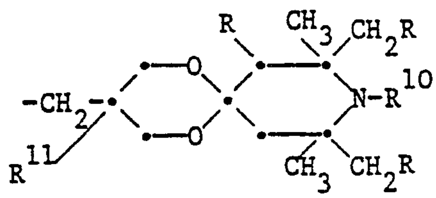

(VII)

and E is a group of the formula VIII

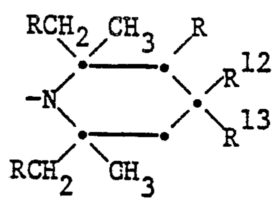

(VIII)

in which

R is hydrogen or methyl,

$R^{10}$ is hydroxyl, $C_1$-$C_{12}$-alkyl, $C_3$-$C_6$-alkenyl, $C_3$-$C_4$-alkynyl, $C_7$-$C_{12}$-phenylalkyl, glycidyl, $C_1$-$C_4$-alkyl which is substituted by halogen, —CN, —$COOR^{15}$ or —$CON(R^{16})(R^{17})$, or a group —CO—$R^{18}$ —CO—$OR^{15}$, —CO—$N(R^{16})(R^{17})$, —$CH_2$—$CH(R^{19})$—$OR^{20}$, —SO—$R^{21}$, —$SO_2$—$R^{21}$, —$OR^{15}$ or —OOC—$R^{18}$,

$R^{11}$ is methyl or ethyl,

$R^{12}$ is hydrogen, —$OR^{22}$, —OOC—$R^{18}$ or —$N(R^9)$—CO—$R^{18}$ and

$R^{13}$ is hydrogen, —CN, —$COOR^{15}$ or —$CONH_2$ or

$R^{12}$ and $R^{13}$ together form a group of the following formulae

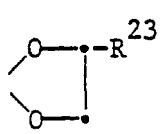

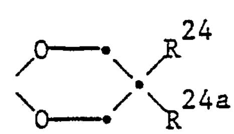

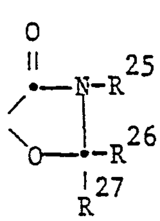

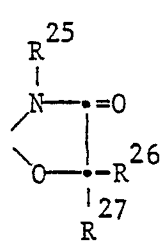

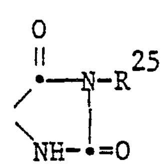

$R^{15}$ is $C_1$-$C_{12}$-alkyl, allyl, benzyl or cyclohexyl,

$R^{16}$ is $C_1$-$C_{12}$-alkyl, allyl, cyclohexyl, benzyl or phenyl, and

$R^{17}$ is hydrogen or $C_1$-$C_8$-alkyl, or

$R^{16}$ and $R^{17}$, together with the N atom, are a 5-membered or 6-membered heterocyclic ring,

$R^{18}$ is hydrogen, $C_1$-$C_{12}$-alkyl, $C_2$-$C_6$-alkenyl, chloromethyl, $C_5$-$C_{12}$-cycloalkyl, $C_7$-$C_{12}$-phenylalkyl, phenyl, $C_7$-$C_{10}$-alkylphenyl or phenyl, phenylmethyl or phenylethyl which is substituted by one or two $C_1$-$C_4$-alkyl groups and a hydroxyl group,

$R^{19}$ is hydrogen, $C_1$-$C_4$-alkyl, $C_2$-$C_{13}$-alkoxymethyl, phenyl or phenoxymethyl,

$R^{20}$ is hydrogen, $C_1$-$C_{12}$-alkyl, —CO—$R^{18}$ or —CO—N($R^{16}$)($R^{17}$),

$R^{21}$ is $C_1$-$C_{12}$-alkyl, phenyl or $C_7$-$C_{18}$-alkylaryl,

$R^{22}$ is hydrogen, $C_1$-$C_{12}$-alkyl, allyl or benzyl,

$R^{23}$ is hydrogen, methyl or ethyl,

$R^{24}$ and $R^{24a}$ independently of one another are H or $C_1$-$C_4$-alkyl,

$R^{25}$ is hydrogen, $C_1$-$C_{12}$-alkyl, $C_3$-$C_6$-alkenyl or $C_7$-$C_{12}$-phenylalkyl,

$R^{26}$ is hydrogen, $C_1$-$C_{12}$-alkyl, $C_3$-$C_8$-cycloalkyl or benzyl, and

$R^{27}$ is $C_1$-$C_{12}$-alkyl, $C_5$-$C_8$-cycloalkyl or phenyl, or

$R^{26}$ and $R^{27}$, together with the C atom to which they are attached, form a $C_5$-$C_{12}$-cycloalkane or $C_5$-$C_{12}$-alkylcycloalkane ring,

or

c) hydrogen, $C_1$-$C_{18}$-alkyl, $C_3$-$C_6$-alkenyl, $C_7$-$C_{12}$-phenylalkyl, $C_3$-$C_{12}$-alkoxyalkyl or $C_3$-$C_{14}$-alkoxycarbonylalkyl,

$R^4$ is hydrogen, $C_1$-$C_{18}$-alkyl, $C_5$-$C_8$-cycloalkyl, a group of the formula II, a group of the formula III or IV or a group —$C_mH_{2m}$—COO($C_1$-$C_4$-alkyl) and

$R_5$ is hydrogen, $C_1$-$C_{18}$-alkyl, $C_5$-$C_8$-cycloalkyl, phenyl, $C_7$-$C_9$-phenylalkyl, a group of the formula II, a group of the formula III or IV or a group of the formula V,

at least one of the radicals $R^1$, $R^2$, $R^3$, $R^4$ and $R^5$ containing a sterically hindered phenol group and at least one of these radicals containing a polyalkylpiperidine group.

3. A colour-photographic recording material according to claim 1, containing at least one compound of the formula I in which X is a group —N($R^3$)— or —C($R^4$)($R^5$)—, and at least one of the radicals $R^1$, $R^2$, $R^3$, $R^4$ and $R^5$ contains a sterically hindered phenol group and at least one of the radicals contains an N-substituted 2,2,6,6-tetramethylpiperidine group.

4. A colour-photographic recording material according to claim 2, containing a compound of the formula I in which X is a group —N($R^3$)— or —C($R^4$)($R^5$)—, and $R^1$, $R^2$ and $R^3$ independently of one another are either

a) a group of the formula IIa or IIb

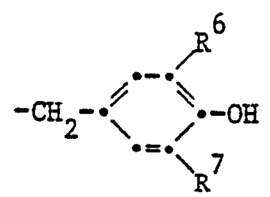
(IIa)

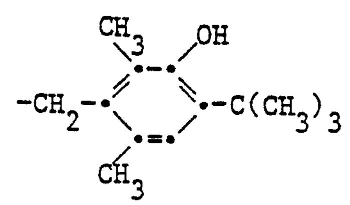
(IIb)

in which

$R^6$ is hydrogen, $C_1$-$C_8$-alkyl, $C_5$-$C_8$-cycloalkyl or $C_7$-$C_9$-phenylalkyl and

$R^7$ is $C_1$-$C_8$-alkyl, $C_5$-$C_8$-cycloalkyl or phenyl,

or

b) a group of the formula III in which

m is 1, 2 or 3,

Y is —O— or —N($R^9$)— in which $R^9$ is hydrogen, $C_1$-$C_{12}$-alkyl or cyclohexyl or $R^9$ is D,

D is a group of the formula V in which R is hydrogen and $R^{10}$ is $C_1$-$C_6$-alkyl, allyl, benzyl, $C_1$-$C_4$-alkyl which is substituted by —CN or —COO$R^{15}$, or a group —CO—$R^{18}$, —COO$R^{15}$, —CON($R^{16}$)($R^{17}$), —$CH_2$—CH($R^{19}$)—O$R^{20}$, —SO$R^{21}$, —$SO_2R^{21}$, —O$R^{15}$ or —OOC—$R^{18}$ in which $R^{15}$ is $C_1$-$C_8$-alkyl, $R^{16}$ is $C_1$-$C_8$-alkyl, cyclohexyl or phenyl and $R^{17}$ is hydrogen or $C_1$-$C_8$-alkyl, or $R^{16}$ and $R^{17}$, together with the N atom, are pyrrolidine, piperidine or morpholine, $R^{18}$ is $C_1$-$C_4$-alkyl, $C_2$-$C_3$-alkenyl, cyclohexyl, phenyl or benzyl, $R^{19}$ is hydrogen or methyl, $R^{20}$ is hydrogen, $C_1$-$C_8$-alkyl or a group of the formula IX

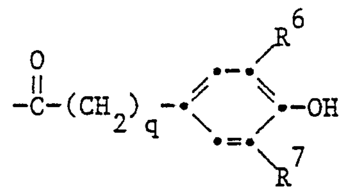

(IX)

in which q is 0, 1 or 2, and $R^{21}$ is $C_1$-$C_4$-alkyl, phenyl or p-tolyl, or

c) hydrogen,

$R^4$ and $R^5$ independently of one another are hydrogen or $C_1$-$C_{12}$-alkyl or are as defined under a) or b), and at least one of the radicals $R^1$, $R^2$, $R^3$, $R^4$ and $R^5$ contains a sterically hindered phenol group and at least one of these radicals contains an N-substituted 2,2,6,6-tetramethylpiperidine group.

5. A colour-photographic recording material according to claim 4, containing a compound of the formula I in which

X is a group —N($R^3$)—,

$R^1$ and $R^2$ are a group of the formula IIa in which $R^6$ is hydrogen or $C_1$-$C_4$-alkyl and $R^7$ is $C_1$-$C_4$-alkyl,

$R^3$ is a group of the formula III in which m is 1 or 2,

Y is —O— or —N($R^9$)— in which $R^9$ is hydrogen or $C_1$-$C_{12}$-alkyl or $R^9$ is D, and

D is a group of the formula V in which R is hydrogen and $R^{10}$ is methyl, allyl, benzyl, acetyl, acryloyl, methoxy, ethoxy or —$CH_2CH_2OR^{20}$ in which $R^{20}$ is hydrogen or a group of the formula IX in which q is 0, 1 or 2 and $R^6$ and $R^7$ are as defined above.

6. A colour-photographic recording material according to claim 4, containing a compound of the formula I in which

X is a group —C($R^4$)($R^5$)—,

$R^1$ and $R^2$ are a group of the formula IIa in which $R^6$ is hydrogen or $C_1$-$C_4$-alkyl and $R^7$ is $C_1$-$C_4$-alkyl,

$R^4$ is a group of the formula III in which m is 1 or 2,

Y is —O— or —N($R^9$)— in which $R^9$ is hydrogen or $C_1$-$C_{12}$-alkyl, or $R^9$ is D, and

D is a group of the formula V in which R is hydrogen and $R^{10}$ is methyl, allyl, benzyl, acetyl, acryloyl, methoxy, acetoxy or —$CH_2CH_2OR^{20}$ in which $R^{20}$ is hydrogen or a group of the formula IX in which q is 0, 1 or 2, $R^6$ is hydrogen or $C_1$-$C_4$-alkyl and $R^7$ is $C_1$-$C_4$-alkyl, and

$R^5$ is as defined for $R^4$ or is $C_1$-$C_{12}$-alkyl or is as defined for $R^1$.

7. A colour-photographic recording material according to claim 4, containing a compound of the formula I in which

X is a group —C($R^4$)($R^5$)—,

$R^1$ and $R^2$ are a group of the formula III in which m is 1 or 2,

Y is —O— or —N($R^9$)— in which $R^9$ is hydrogen or $C_1$-$C_{12}$-alkyl, or $R^9$ is D, and

D is a group of the formula V in which R is hydrogen and $R^{10}$ is methyl, allyl, benzyl, acetyl, acryloyl, metoxy, (sic) acetoxy or —$CH_2CH_2OR^{20}$ in which $R^{20}$ is hydrogen or a group of the formula IX in which q is 0, 1 or 2, $R^6$ is hydrogen or $C_1$-$C_4$-alkyl and $R^7$ is $C_1$-$C_4$-alkyl,

$R^4$ is a group of the formula IIa in which $R^6$ and $R^7$ are as defined above and

$R^5$ is as defined for $R^4$ or is $C_1$-$C_{12}$-alkyl or is as defined for $R^1$.

8. A colour-photographic recording material according to claim 1, which, in addition to a stabiliser of the formula I contains a light stabiliser belonging to the class comprising the ultraviolet absorbers.

9. A colour-photographic recording material according to claim 1, which contains 1 to 2 000, preferably 100 to 800 mg per $m^2$ of a compound of the formula I.

10. The use of the compounds of the formula I defined in claim 1 as stabilisers for colour-photographic recording materials.

## Revendications

1. Matière pour enregistrement photographique en couleurs qui contient comme stabilisant, dans au moins une couche d'émulsion d'halogénure d'argent photosensible, une couche intermédiaire, une couche réceptrice d'image et/ou une couche protectrice, au moins un composé répondant à la formule I :

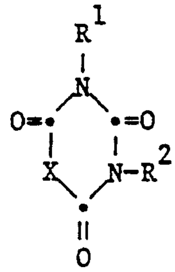

(I)

dans laquelle X représente un radical

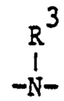 ou 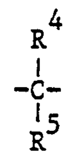

et $R^1$, $R^2$, $R^3$, $R^4$ et $R^5$ représentent des radicaux organiques univalents dont un au moins contient un radical de phénol à empêchement stérique et un au moins contient un radical de polyalkyl-pipéridine.

2. Matière pour enregistrement photographique en couleurs selon la revendication 1, qui contient au moins un composé de formule I dans lequel : X représente un radical $—N(R^3)—$ ou $—C(R^4)(R^5)—$, $R^1$, $R^2$ et $R^3$ représentent chacun, indépendamment les uns des autres, soit :

a) un radical répondant à la formule II :

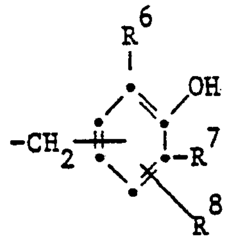

(II)

dans laquelle :

$R^6$ représente l'hydrogène, un alkyle en $C_1$-$C_8$, un cycloalkyle en $C_5$-$C_8$, un phénylalkyle en $C_7$-$C_9$, un phényle ou un alkylphényle en $C_7$-$C_{10}$,

$R^7$ représente un alkyle en $C_1$-$C_8$, un cycloalkyle en $C_5$-$C_8$, un phénylalkyle en $C_7$-$C_9$, un phényle ou un alkylphényle en $C_7$-$C_{10}$ et

$R^8$ représente l'hydrogène ou un méthyle,

soit

b) un radical répondant à l'une des formules III et IV :

$$—C_mH_{2m}—CO—Y—D \qquad (III)$$

$$—C_mH_{2m}—CO—O—C_nH_{2n}—E \qquad (IV)$$

dans lesquelles :

m représente un nombre de 1 à 4,

n représente un nombre de 2 à 5,

Y représente —O— ou un radical $—NR^9—$ dans lequel le symbole $R^9$ représente l'hydrogène, un alkyle en $C_1$-$C_{12}$, un cycloalkyle en $C_5$-$C_{12}$, un alcoxyalkyle en $C_3$-$C_{12}$, un dialkylamino-alkyle en $C_4$-$C_{12}$ ou un radical :

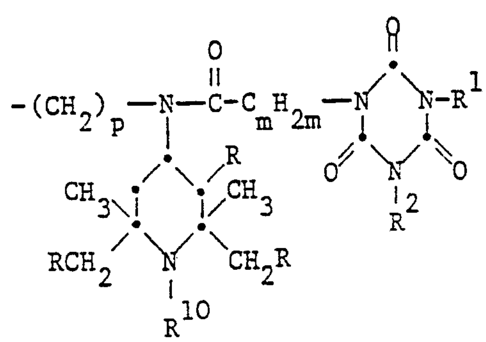

dans lequel p désigne un nombre de 2 à 12, ou $R^9$ a la signification de D,

D représente un radical répondant à l'une des formules V, VI et VII

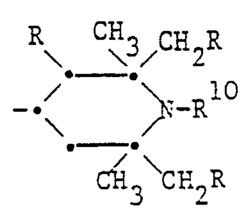

(V)

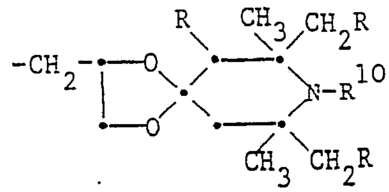

(VI)

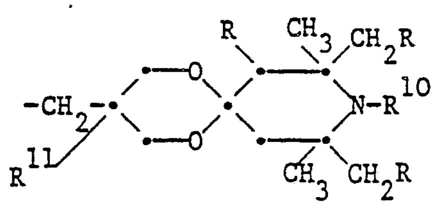

(VII)

et E représente un radical de formule VIII :

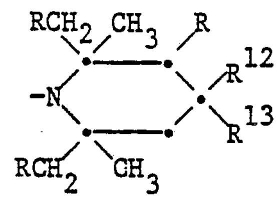

(VIII)

formules dans lesquelles :

R représente l'hydrogène ou un méthyle,

$R^{10}$ représente un hydroxy, un alkyle en $C_1$-$C_{12}$, un alcényle en $C_3$-$C_6$, un alcynyle en $C_3$ ou $C_4$, un phénylalkyle en $C_7$-$C_{12}$, un glycidyle, un alkyle en $C_1$-$C_4$ porteur d'un halogène ou d'un radical —CN, —$COOR^{15}$ ou —$CON(R^{16})(R^{17})$, ou représente un radical —CO—$R^{18}$, —CO—$OR^{15}$, —CO—$N(R^{16})(R^{17})$, —$CH_2$—$CH(R^{19})$—$OR^{20}$, —SO—$R^{21}$, —$SO_2$—$R^{21}$, —$OR^{15}$ ou —OOC—$R^{18}$,

$R^{11}$ représente un méthyle ou un éthyle,

$R^{12}$ représente l'hydrogène ou un radical —$OR^{22}$, —OOC—$R^{18}$ ou —$N(R^9)$—CO—$R^{18}$ et

$R^{13}$ l'hydrogène ou un radical —CN, —$COOR^{15}$ ou —$CONH_2$, ou

$R^{12}$ et $R^{13}$ forment ensemble un radical répondant à l'une des formules suivantes :

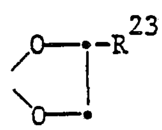

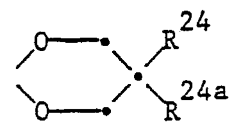

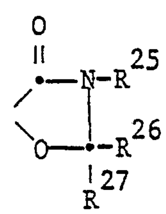

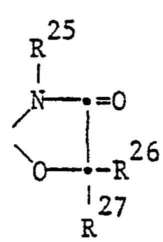

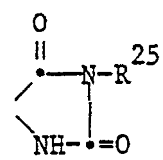

$R^{15}$ représente un alkyle en $C_1$-$C_{12}$, un allyle, un benzyle ou un cyclohexyle,

$R^{16}$ représente un alkyle en $C_1$-$C_{12}$, un allyle, un cyclohexyle, un benzyle ou un phényle et

$R^{17}$ l'hydrogène ou un alkyle en $C_1$-$C_8$, ou

$R^{16}$ et $R^{17}$ forment ensemble, et avec l'atome d'azote, un hétérocycle à 5 ou 6 maillons,

$R^{18}$ représente l'hydrogène, un alkyle en $C_1$-$C_{12}$, un alcényle en $C_2$-$C_6$, un chlorométhyle, un cycloalkyle en $C_5$-$C_{12}$, un phénylalkyle en $C_7$-$C_{12}$, un phényle, un alkylphényle en $C_7$-$C_{10}$ ou un radical phényle, phénylméthyle ou phényléthyle, porteur d'un ou de deux alkyles en $C_1$-$C_4$ et d'un hydroxy,

$R^{19}$ représente l'hydrogène, un alkyle en $C_1$-$C_4$, un alcoxyméthyle en $C_2$-$C_{13}$, un phényle ou un phénoxyméthyle,

$R^{20}$ représente l'hydrogène, un alkyle en $C_1$-$C_{12}$, un radical —CO—$R^{18}$ ou un radical —CO—$N(R^{16})(R^{17})$,

$R^{21}$ représente un alkyle en $C_1$-$C_{12}$, un phényle ou un alkylaryle en $C_7$-$C_{18}$,
$R^{22}$ représente l'hydrogène, un alkyle en $C_1$-$C_{12}$, un allyle ou un benzyle,
$R^{23}$ représente l'hydrogène, un méthyle ou un éthyle,
$R^{24}$ et $R^{24a}$ représentent chacun, indépendamment l'un de l'autre, H ou un alkyle en $C_1$-$C_4$,
$R^{25}$ représente l'hydrogène, un alkyle en $C_1$-$C_{12}$, un alcényle en $C_3$-$C_5$ ou un phénylalkyle en $C_7$-$C_{12}$,
$R^{26}$ représente l'hydrogène, un alkyle en $C_1$-$C_{12}$, un cycloalkyle en $C_3$-$C_8$ ou un benzyle et
$R^{27}$ un alkyle en $C_1$-$C_{12}$, un cycloalkyle en $C_5$-$C_8$ ou un phényle, ou
$R^{26}$ et $R^{27}$ forment ensemble, et avec l'atome de carbone auquel ils sont liés, un noyau de cycloalcane en $C_5$-$C_{12}$ ou d'alkylcycloalcane,
soit

c) l'hydrogène, un alkyle en $C_1$-$C_{18}$, un alcényle en $C_3$-$C_6$, un phénylalkyle en $C_7$-$C_{12}$, un alcoxyalkyle en $C_3$-$C_{12}$ ou un alcoxycarbonylalkyle en $C_3$-$C_{14}$,

$R^4$ représente l'hydrogène, un alkyle en $C_1$-$C_{18}$, un cycloalkyle en $C_5$-$C_8$, un radical de formule II, un radical de formule III ou IV ou un radical $—C_mH_{2m}—COO$(alkyl en $C_1$-$C_4$) et

$R^5$ représente l'hydrogène, un alkyle en $C_1$-$C_{18}$, un cycloalkyle en $C_5$-$C_8$, un phényle, un phénylalkyle en $C_7$-$C_9$, un radical de formule II, un radical de formule III ou IV ou un radical de formule V, au moins un des radicaux $R^1$, $R^2$, $R^3$, $R^4$ et $R^5$ contenant un radical de phénol à empêchement stérique, et au moins un de ces radicaux contenant un radical de polyalkyl-pipéridine.

3. Matière pour enregistrement photographique en couleurs selon la revendication 1, matière qui contient au moins un composé de formule I dans laquelle X représente un radical $—N(R^3)—$ ou $—C(R^4)(R^5)—$ et, parmi les radicaux $R^1$, $R^2$, $R^3$, $R^4$ et $R^5$, au moins un contient un radical de phénol à empêchement stérique et au moins un contient un radical de tétraméthyl-2,2,6,6 pipéridine substitué à l'azote.

4. Matière pour enregistrement photographique en couleurs selon la revendication 2, qui contient un composé de formule I dans lequel :

X représente un radical $—N(R^3)—$ ou $—C(R^4)(R^5)—$, $R^1$, $R^2$ et $R^3$ représentent chacun, indépendamment les uns des autres, soit :

a) un radical répondant à l'une des formules IIa et IIb :

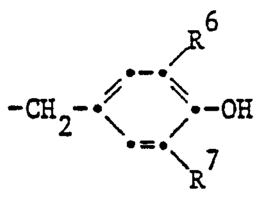

(IIa)

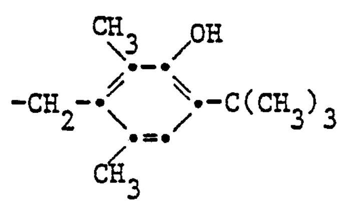

(IIb)

où :

$R^6$ représente l'hydrogène, un alkyle en $C_1$-$C_8$, un cycloalkyle en $C_5$-$C_8$ ou un phénylalkyle en $C_7$-$C_9$, et

$R^7$ représente un alkyle en $C_1$-$C_8$, un cycloalkyle en $C_5$-$C_8$ ou un phényle,
soit

b) un radical de formule III dans lequel :

m représente un nombre égal à 1, à 2 ou à 3,

Y représente —O— ou un radical $—N(R^9)—$ dans lequel $R^9$ désigne l'hydrogène, un alkyle en $C_1$-$C_{12}$ ou un cyclohexyle ou a la signification de D, et

D représente un radical de formule V dans lequel R représente l'hydrogène et $R^{10}$ un alkyle en $C_1$-$C_6$, un allyle, un benzyle, un alkyle en $C_1$-$C_4$ porteur d'un radical —CN ou $—COOR^{15}$, ou un radical $—CO—R^{18}$, $—COOR^{15}$, $—CON(R^{16})(R^{17})$, $—CH_2—CH(R^{19})—OR^{20}$, $—SO—R^{21}$, $SO_2—R^{21}$, $—OR^{15}$ ou $—OOC—R^{18}$, radicaux dans lesquels $R^{15}$ représente un alkyle en $C_1$-$C_{18}$, $R^{16}$ un alkyle en $C_1$-$C_8$, un cyclohexyle ou un phényle, $R^{17}$ l'hydrogène ou un alkyle en $C_1$-$C_8$, ou $R^{16}$ et $R^{17}$ forment ensemble, et avec l'atome d'azote, un cycle de pyrrolidine, de pipéridine ou de morpholine, $R^{18}$ représente un alkyle en $C_1$-$C_4$, un alcényle en $C_2$ ou $C_3$, un cyclohexyle, un phényle ou un benzyle, $R^{19}$ l'hydrogène ou un méthyle et $R^{20}$ l'hydrogène, un alkyle en $C_1$-$C_8$, ou un radical de formule IX :

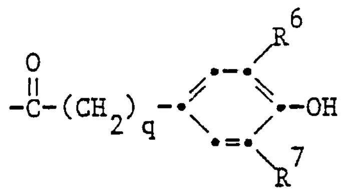

(IX)

dans lequel q est égal à 0, à 1 ou à 2, et $R^{21}$ représente un alkyle en $C_1$-$C_4$, un phényle ou un p-tolyle, soit c) l'hydrogène,

$R^4$ et $R^5$ représentent chacun, indépendamment l'un de l'autre, l'hydrogène ou un alkyle en $C_1$-$C_{12}$ ou ont chacun, indépendamment l'un de l'autre, l'une des significations qui ont été données sous a) ou b), et, parmi les radicaux $R^1$, $R^2$, $R^3$, $R^4$ et $R^5$, au moins un contient un radical de phénol à empêchement stérique et au moins un radical de tétraméthyl-2,2,6,6 pipéridine substitué à l'azote.

5. Matière pour enregistrement photographique en couleurs selon la revendication 4, qui contient un composé de formule I dans lequel :

X représente un radical —N($R^3$)—,

$R^1$ et $R^2$ représentent chacun un radical de formule IIa dans lequel $R^6$ désigne l'hydrogène ou un alkyle en $C_1$-$C_4$ et $R^7$ un alkyle en $C_1$-$C_4$, et

$R^3$ représente un radical de formule III dans lequel :

m désigne un nombre égal à 1 ou à 2,

Y représente —O— ou un radical —N($R^9$)— dans lequel $R^9$ représente l'hydrogène ou un alkyle en $C_1$-$C_{12}$ ou encore a la signification de D, et

D représente un radical de formule V dans lequel R représente l'hydrogène et $R^{10}$ un radical méthyle, allyle, benzyle, acétyle, acryloyle, méthoxy ou éthoxy ou un radical —$CH_2CH_2OR^{20}$ dans lequel $R^{20}$ représente l'hydrogène ou un radical de formule IX dans lequel q est égal à 0, à 1 ou à 2 tandis que $R^6$ et $R^7$ ont les significations qui leur ont été données ci-dessus.

6. Matière pour enregistrement photographique en couleurs selon la revendication 4, qui contient un composé de formule I dans lequel :

X représente un radical —C($R^4$)($R^5$)—,

$R^1$ et $R^2$ représentent chacun un radical de formule IIa dans lequel $R^6$ désigne l'hydrogène ou un alkyle en $C_1$-$C_4$ et $R^7$ un alkyle en $C_1$-$C_4$,

$R^4$ représente un radical de formule III dans lequel m désigne le nombre 1 ou le nombre 2,

Y représente —O— ou un radical —N($R^9$)— dans lequel $R^9$ représente l'hydrogène ou un alkyle en $C_1$-$C_{12}$ ou encore a la signification de D, et

D représente un radical de formule V dans lequel R représente l'hydrogène et $R^{10}$ un radical méthyle, allyle, benzyle, acétyle, acryloyle, méthoxy ou acétoxy ou un radical —$CH_2CH_2OR^{20}$ dans lequel $R^{20}$ représente l'hydrogène ou un radical de formule IX dans lequel q est égal à 0, à 1 ou à 2, $R^6$ représente l'hydrogène ou un alkyle en $C_1$-$C_4$ et $R^7$ représente un alkyle en $C_1$-$C_4$, et

$R^5$ a la même signification que $R^4$ ou représente un alkyle en $C_1$-$C_{12}$ ou encore a la même signification que $R^1$.

7. Matière pour enregistrement photographique en couleurs selon la revendication 4, qui contient un composé de formule I dans lequel :

X représente un radical —C($R^4$)($R^5$)—,

$R^1$ et $R^2$ représentent chacun un radical de formule III dans lequel :

m désigne un nombre égal à 1 ou à 2,

Y représente —O— ou un radical —N($R^9$)— dans lequel $R^9$ représente l'hydrogène ou un alkyle en $C_1$-$C_{12}$ ou a la signification de D,

D représente un radical de formule V dans lequel R représente l'hydrogène et $R^{10}$ un radical méthyle, allyle, benzyle, acétyle, acryloyle, méthoxy ou acétoxy ou un radical —$CH_2CH_2OR^{20}$ dans lequel $R^{20}$ représente l'hydrogène ou un radical de formule IX dans lequel q est égal à 0, à 1 ou à 2, $R^6$ représente l'hydrogène, ou un alkyle en $C_1$-$C_4$ et $R^7$ représente un alkyle en $C_1$-$C_4$,

$R^4$ représente un radical de formule IIa dans lequel $R^6$ et $R^7$ ont les significations précédemment données et

$R^5$ a la même signification que $R^4$ ou représente un alkyle en $C_1$-$C_{12}$ ou a la même signification que $R^1$.

8. Matière pour enregistrement photographique en couleurs selon la revendication 1, caractérisée en ce qu'elle contient, en plus d'un stabilisant de formule I, un stabilisant à la lumière de la catégorie des absorbeurs de rayons ultra-violets.

9. Matière pour enregistrement photographique en couleur selon la revendication 1, caractérisée en ce qu'elle contient, par mètre carré, de 1 à 2 000 mg d'un composé de formule I, de préférence de 100 à 800 mg.

10. Application des composés de formule I qui ont été définis à la revendication 1 comme stabilisants pour des matières devant servir à l'enregistrement photographique en couleurs.